# EUROPÄISCHE PATENTSCHRIFT

(11) **EP 3 318 298 B1**
(45) Veröffentlichungstag und Bekanntmachung des Hinweises auf die Patenterteilung: **29.12.2021**
(21) Anmeldenummer: 17200301.4
(22) Anmeldetag: 07.11.2017
(51) Int. Cl.: A61M 16/20

(54) **EXPIRATIONSVENTIL**
EXPIRATION VALVE
SOUPAPE D'EXPIRATION

(30) Priorität: 08.11.2016 DE 102016121379
(43) Veröffentlichungstag der Anmeldung: 09.05.2018
(73) Patentinhaber: Hamilton Medical AG, 7402 Bonaduz (CH)
(72) Erfinder: Breitruck, Felix, 9470 Buchs SG (CH); De-Stefani, Dino, 7000 Chur (CH)
(74) Vertreter: Schmitt-Nilson Schraud Waibel Wohlfrom Patentanwälte Partnerschaft mbB

(56) Entgegenhaltungen:
- WO-A1-00/45883
- WO-A1-2015/127994
- US-A- 4 699 137
- US-A- 5 005 568
- US-A1- 2009 293 878

## Beschreibung

Die vorliegende Erfindung betrifft ein Expirationsventil eines Beatmungsgeräts, aufweisend einen Ventilkörper, in dem ein Fluideinlass und ein Fluidauslass für Atemluft eines Patienten ausgebildet sind, wobei der Ventilkörper wenigstens eine um den Fluideinlass herum ausgebildete Versteifungsrippe aufweist.

Dieses Expirationsventil wird bei einem Beatmungsgerät zur maschinellen Beatmung von Patienten eingesetzt. Die vorliegende Erfindung betrifft auch ein sol - ches Beatmungsgerät. Die Erfindung ist durch die beigefügten Ansprüche definiert.

Bei der maschinellen Beatmung wird einem Patienten Atemluft auf maschinellem Wege, in der Regel unter einem Überdruck zugeführt. Die maschinelle Beatmung kann die eigene Atmung des Patienten dabei unterstützen oder vollständig er - setzten. Das Zuführen der Beatmungsluft erfolgt in aufeinander folgenden Beat - mungszyklen. Jeder Beatmungszyklus hat eine Inspirationsphase mit nachfolgender Expirationsphase. Dabei wird die Beatmungsluft während der Inspirationsphase eines jeweiligen Beatmungszyklus zugeführt, in der Regel maschinell oder jedenfalls maschinell unterstützt, und mit einem Überdruck gegenüber dem in den Atemwegen des Patienten herrschenden Druck. Das Ausatmen erfolgt während einer nachfolgenden Expirationsphase des Beatmungszyklus. Hierbei erfolgt in der Regel keine Beaufschlagung der Atemwege mit Überdruck oder Unterdruck. Vielmehr soll das Ausatmen passiv erfolgen durch Entspannen der Atemwege ge - genüber dem Umgebungsdruck, wobei eigene Ausatmungsanstrengungen des Patienten soweit als möglich unterstützt werden sollen. Das Ausatmen der Atemluft und die Abgabe der Atemluft an die Umgebung soll mit Hilfe des hier be - schriebenen Expirationsventils erfolgen. Das Expirationsventil ist im Beatmungssystem in einer Leitung für ausgeatmete Luft angeordnet, in der Regel am strom - abwärts liegenden Ende dieser Leitung. Auf seiner vom Patienten abgewandten Seite herrscht in der Regel Umgebungsdruck. Wird es während einer Expirations - phase mit Überdruck gegenüber diesem Druck von der Seite des Patienten her beaufschlagt, so soll das Expirationsventil öffnen und eine Abgabe der ausgeat - meten Luft an die Umgebung ermöglichen. Das Expirationsventil soll dabei Be - ginn und Ende von Expirationsphasen zuverlässig zu erkennen und entspre - chend reagieren. Hierzu dient eine Ventilmembran, die je nachdem, ob auf ihrer den Atemwegen zugewandten Seite ein genügend großer Überdruck gegenüber ihrer der Umgebung zugewandten Seite herrscht, einen Fluiddurchgang zwischen den Atemwegen der Umgebung freigibt oder verschließt.

WO 00/45883 A1 zeigt ein Beatmungsventil mit einem Einlass, durch den Atemluft von einem Beatmungsgerät in das Beatmungsventil einleitbar ist, einem ersten Auslass, durch den Atemluft zu einem Patienten ausgeleitet und Ausatemluft von einem Patienten eingeleitet wird, einem zweiten Auslass, durch den die Ausatemluft des Patienten in einen Hohlraum des Beatmungsventils eingeleitet wird, und Öffnungen, die in dem Hohlraum angeordnet sind, und durch die die Ausatemluft dann aus dem Beatmungsventil ausgeleitet wird. Eine Membran trennt den zweiten Auslass von dem Hohlraum, so dass bei einer Beatmung die Atemluft von dem Beatmungsgerät nicht über die Öffnungen aus dem Beatmungsventil entweicht.

WO 2015/127994 A1 zeigt einen Durchflussmesser eines Beatmungsgeräts mit einem in einem Gehäuse zentral angeordneten Rohr. Das Rohr hat einen Einlassbereich und einen Auslassbereich. Das Gehäuse weist ferner einen Ausleitkanal auf, welcher mit dem Auslassbereich des Rohrs verbunden ist. Eine flexible Membran ist über dem Auslassbereich angeordnet und verschließt diesen.

US 5 005 568 A zeigt ein Isolationsventil, welches in Beatmungsgeräten zum Einsatz kommt. Das Isolationsventil weist ein Einlassrohr und ein Auslassrohr auf, welche durch eine Membran voneinander getrennt sind. Die Membran weist einen Port auf, der bei einer Beatmung eines Patienten Luft von dem Einlassrohr zum Auslassrohr passieren lässt. Beim Ausatmen des Patienten verschließt die Membran das Einlassrohr und die Ausatemluft des Patienten wird von der Membran zu mehreren, im Querschnitt kleineren Auslassöffnungen geleitet.

US 2009/0293878 A1 zeigt eine Beatmungsvorrichtung zum Beatmen eines Patienten durch einen Luftschlauch. Die Beatmungsvorrichtung weist ein Verbindungsrohr zum Patienten auf, durch den Atemluft dem Patienten wird und Ausatemluft vom Patienten abgeführt wird. Dies geschieht durch mehrere kleinere Öffnungen, die um das Verbindungsrohr angeordnet sind.

In der Vergangenheit trat häufig das Problem auf, dass das Expirationsventil im Betrieb unangenehm laute Geräusche verursachte. In manchen Fällen wurde sogar beobachtet, dass vom Expirationsventil oszillierende Druckschwankungen in der ausgeatmeten Luft verursacht oder verstärkt wurden, die letztendlich zu Fehlsteuerungen in der Beatmung führten. Bei diesem als Autotrigger oder Autocycle bekannten Phänomen werden vom Expirationsventil verursachte oder verstärkte Druckschwankungen vom Beatmungsgerät fälschlicherweise als Ende der Expirationsphase detektiert und das Beatmungsgerät leitet vorzeitig die nächste Inspirationsphase ein. Dies ist für den zu beatmenden Patienten sehr unangenehm und kann sogar zur Gefährdung des beatmeten Patienten führen. Das erfindungsgemäße Expirationsventil soll derartige Probleme vermeiden oder jedenfalls verringern.

Gegenüber den bisher verwendeten Expirationsventilen erlaubt das eingangs umschriebene erfindungsgemäße Expirationsventil eine verbesserte Führung der ausgeatmeten Luft. Insbesondere reagiert das Expirationsventil feinfühliger auf den Beginn und das Ende der Expirationsphase, und die Geräuschentwicklung im Betrieb des Expirationsventils ist deutlich unterdrückt. Außerdem gelingt es, das Phänomen des Autotrigger oder Autocycle besser zu beherrschen.

Das Expirationsventil ist so ausgebildet, dass Atemluft beim Ausatmen, also während der Expirationsphase, durch den nunmehr geöffneten Fluideinlass in das Expirationsventil, insbesondere in ein vom Expirationsventil umschlossenes Ventilplenum, eindringen kann. Während der Inspirationsphase ist der Fluideinlass vorzugsweise verschlossen.

Der Fluidauslass ist so ausgebildet, dass ausgeatmete Atemluft in eine Umgebung abgebbar ist.

Es wird vorgeschlagen, den Ventilkörper mit wenigstens einer um den Fluideinlass herum ausgebildeten Versteifungsrippe zu versehen. Der Ventilkörper kann dabei insbesondere mit mehreren um den Fluideinlass herum ausgebildeten Versteifungsrippen versehen sein. Die mehreren Versteifungsrippen können insbesondere in bestimmten Abständen zueinander um einen Umfang des Fluideinlasses herum aufeinander folgen. Ein einzige Versteifungsrippe kann beispielsweise als ein den Fluideinlass wenigstens teilweise umgebender Vollkörper ausgebildet sein. Die wenigstens eine Versteifungsrippe ist insbesondere auf einer vom Fluidströmungsweg abgewandten Seite des Ventilkörpers ausgebildet, so dass sie die Fluidströmung zwischen dem Fluideinlass und dem Fluidauslass nicht stört. Die Versteifungsrippe(n) befindet sich insbesondere auf einer Unterseite des Ventilkörpers, und ist beispielsweise an der Unterseite eines den Boden des Ventilkörpers bildenden Ventilbodens auf einer vom Fluidströmungsweg abgewandten Seite des Ventilbodens ausgebildet.

Die Versteifungsrippe bzw. die Versteifungsrippen sind geeignet, den Ventilboden zu versteifen. Dies macht sich besonders vorteilhaft bemerkbar in einem sich auswölbenden Bereich, der den Fluideinlass umgibt. Ein solcher sich auswölbender Bereich kann eine um den Fluideinlass herum führende Rinne bilden, wie dies nachfolgend noch näher beschrieben wird. Vorsehen von Versteifungsrippe(n) am Ventilkörper führt zu einer Erhöhung des Flächenträgheitsmoments des Ventilkörpers, insbesondere des Ventilbodens, über den Querschnitt des Fluidströmungswegs hinweg. Der Ventilkörper wird in diesem Bereich verwindungsstreifer, so dass die in das Ventilplenum einströmende und entlang des Fluidströmungswegs strömende Fluidströmung weniger Schwingungen des Ventilkörpers, insbesondere des Ventilbodens, anregt. Dadurch wird vor allem Geräuschentwicklung unterdrückt, es entstehen aber auch viel weniger Druck-Rückwirkungen, die sich in der am Fluideinlass angeschlossenen Atemwegsleitung bemerkbar machen. Das Problem fehlerhafter Erkennung des Endes eines Expirationszyklus und dadurch ausgelöster Autotrigger oder Autocycle Vorgänge im Beatmungsgerät kann signifikant unterdrückt werden.

Der Ventilkörper kann insbesondere aus Kunststoffmaterial hergestellt sein. Es bietet sich beispielsweise an, den Ventilkörper im Spritzgussverfahren herzustellen. Dann ist der Ventilkörper ein Spritzgussteil. Beispielsweise kann der Ventilkörper einschließlich des Ventilbodens aus einem spritzgussfähigen Kunststoffmaterial hergestellt sein, etwa Polyethylen, Polypropylen oder einem anderen thermoplastischen Kunststoffmaterial.

Es ist günstig, wenn die wenigstens eine Versteifungsrippe integral mit dem Ventilkörper ausgebildet ist. Integral ist so verstehen, dass die wenigstens eine Versteifungsrippe und der Ventilkörper als ein Bauteil hergestellt sind, beispielsweise dass die wenigstens eine Versteifungsrippe im selben Spritzgussprozess hergestellt ist wie der Ventilkörper, insbesondere als Einkomponentenspritzgussteil.

Günstigerweise kann die wenigstens eine Versteifungsrippe bezogen auf einen Rand des Fluideinlasses nach außen weg verlaufen. Nach außen weg soll dabei die Richtung der wenigstens einen Versteifungsrippe bezeichnen, die so angeordnet ist, dass sie den Bereich des Ventilbodens zwischen dem Rand des Fluideinlasses und dem äußeren Rand des Ventilbodens möglichst effektiv versteift. Insbesondere hat die wenigstens eine Versteifungsrippe so einen Verlauf quer zu einem um den Fluideinlass herum führenden Fluidströmungsweg. Insbesondere kann die wenigstens eine Versteifungsrippe bezüglich einer Mitte eines Querschnitts des Fluideinlasses in radialer Richtung verlaufen. Es ist aber auch denkbar, dass die wenigstens eine Versteifungsrippe nicht genau in radialer Richtung verläuft, sondern zusätzlich in Umfangsrichtung, beispielsweise spiralförmig auf den äußeren Rand des Ventilbodens zu. Insbesondere kann die wenigstens eine Versteifungsrippe außerhalb des Rands des Fluideinlasses angeordnet sein.

In Ausführungsformen bildet der Ventilkörper ein topfförmiges Ventilplenum, welches wenigstens zum Teil an seiner Unterseite von dem Ventilkörper, insbesondere von einem vom Ventilkörper gebildeten Ventilboden und ggf. von einem vom Ventilboden ausgehenden und ebenfalls vom Ventilkörper gebildeten äußeren Rand, umschlossen ist und in das der Fluideinlass mündet. An seiner Oberseite kann das Ventilplenum von einer einen Deckel bildenden Ventilmembran begrenzt sein. Das Ventilplenum bildet einen Hohlraum, der über den Fluid einlass mit Fluid (ausgeatmete Atemluft) beaufschlagbar ist und aus dem über den Fluidauslass das Fluid (ausgeatmete Atemluft) ausströmen kann. Insbesondere ist in dem Ventilplenum der Fluidströmungsweg zwischen dem Fluideinlass und dem Fluidauslass gebildet. Insbesondere ist das Ventilplenum wenigstens zum Teil an seiner Unterseite von dem Ventilboden begrenzt und der Fluideinlass mündet in den Ventilboden. Der Ventilboden kann sich dabei insbesondere tellerartig zwischen dem Fluideinlass und einem äußeren Rand des Ventilkörpers (insbesondere des Ventilplenums) erstrecken, wobei der äußere Rand des Ventilbodens zugleich den äußeren Rand des Ventilkörpers bildet. Mit dem Begriff "tellerartig" soll umschrieben sein, dass der Ventilboden sich, abgesehen von einer möglichen noch näher zu erläuternden rinnenartigen Vertiefung um den Fluideinlass herum, im Wesentlichen flach oder flach konisch zwischen dem Fluideinlass und einem äußeren Rand erstreckt. Der Fluideinlass ist dann insbesondere in einem zentralen Teil des Ventilbodens ausgebildet. Der Fluideinlass kann insbesondere einen kreisförmigen Querschnitt aufweisen mit ebenfalls kreisförmiger Fluideintrittsfläche.

In bestimmten Ausführungsformen kann vorgesehen sein, dass der Ventilboden sich in einem Bereich um den Fluideinlass herum in axialer Richtung auswölbt. Mit der Formulierung "in axialer Richtung auswölbt" soll dabei ausgedrückt werden, dass der Ventilboden in wenigstens einem eine Fluideinlassachse enthaltenden Schnitt einen konvexen Verlauf hat, und zwar in einem zwischen dem Rand des Fluideinlasses und dem Außenrand des Ventilbodens liegenden Bereich. Konvex bedeutet, dass in einem solchen Schnitt eine zwei Punkte auf dem Ventilboden verbindende Linie durch das Ventilplenum verläuft. Der Ventilboden bildet demgemäß eine um den Fluideinlass umlaufende Rinne aus, deren Boden sich zwischen dem Rand des Fluideinlasses und dem Außenrand des Ventilbodens in axialer Richtung auswölbt. Im Gegensatz dazu ist der Ventilboden bei derzeit Verwendung findenden Expirationsventilen flach konisch ausgebildet, er bildet also keine Auswölbung.

Die wenigstens eine Versteifungsrippe ist geeignet, den Ventilboden in dem sich auswölbenden Bereich zu versteifen. Dadurch lässt sich Geräuschentwicklung bei strömender Atemluft verringern, weil Schwingungen des Ventilbodens unterdrückt werden.

Die wenigstens eine Versteifungsrippe kann wenigstens eine erste Versteifungsrippe und wenigstens eine zweite Versteifungsrippe umfassen. Die wenigstens eine erste Versteifungsrippe ist eine innere Versteifungsrippe, die radial innerhalb der wenigstens einen zweiten Versteifungsrippe angeordnetist. Die wenigstens eine zweite Versteifungsrippe ist demgemäß eine äußere Versteifungsrippe. Die wenigstens eine erste Versteifungsrippe verläuft insbesondere von dem Rand des Fluideinlasses entlang des Ventilbodens nach außen. Die wenigstens eine zweite Versteifungsrippe verläuft insbesondere von einem äußeren Rand des Ventilbodens entlang des Ventilbodens nach innen. Die wenigstens eine erste Versteifungsrippe verläuft insbesondere zwischen dem Rand des Fluideinlasses und dem sich auswölbenden Bereich des Ventilbodens. Die wenigstens eine zweiten Versteifungsrippe verläuft insbesondere zwischen dem sich auswölbenden Bereich des Ventilbodens und einem äußeren Rand des Ventilbodens. Insbesondere ender die wenigstens eine zweite Versteifungsrippe in einem am äußeren Rand des Ventilbodens ausgebildeten flanschartigen Ansatz. Der Ansatz kann beispielsweise mindestens einen um den Rand des Ventilbodens umlaufenden Absatz aufweisen, vorzugsweise mindestens zwei im Abstand zueinander angeordnete Absätze, wobei die wenigstens eine zweite Versteifungsrippe in den Absatz, bzw. einen inneren der Absätze, übergeht.

Insbesondere kann der Ventilboden von einem eine Fluideintrittsfläche definierenden Rand des Fluideinlasses ausgehen. Es entsteht kein Absatz zwischen der Fluideintrittsfäche und dem Ventilboden. Auf diese Weise werden scharfkantige Begrenzungen des Fluideinlasses gegenüber dem Ventilplenum vermieden. Das fördert die Ausbildung einer möglichst ruhigen Strömung des Fluids in das Expirationsventil sowie durch das Expirationsventil während der Expirationsphase. Bei bekannten Expirationsventilen ragt demgegenüber der Fluideinlass in das Ventilplenum hinein, es steht insbesondere der Fluideinlass ein Stück weit über den Ventilboden vor. Der Ventilboden geht dann nicht von dem die Fluideintrittsfläche definierenden Rand des Fluideinlasses aus und die Fluidströmung passiert einen Absatz beim Einströmen in das Ventilplenum, was die Ausbildung von Turbulenz und Strömungsabrissen in der in das Expirationsventil einströmenden Fluidströmung fördert.

Zur Reduzierung von Geräuschbildung und Druck-Rückwirkungen kann auch betragen, wenn das Verntilplenum entlang des Fluidströmungswegs zwischen Fluideinlass und Fluidauslass möglichst strömungsgünstig ausgelegt ist. Die strömungsgünstige Form des Ventilplenums, insbesondere des Fluidströmungswegs kann insbesondere verbessert werden, wenn der Ventilboden ausgehend von einer Innenseite des Fluideinlasses am Rand der Fluideintrittsfläche auf seiner dem Ventilplenum zugewandten Seite eine abgerundete Kontur aufweist. Die vom Fluideinlass her in das Ventilplenum einströmende Fluidströmung gelangt so weitgehend unverwirbelt und ohne Strömumgsabrisse in den Fluidströmungsweg und strömt dem Fluidauslass zu. Dies vermindert Betriebsgeräusche bei geöffnetem Fluidauslass unmittelbar. Außerdem werden Druck-Rückschlage in die an dem Fluideinlass angeschlossene Atemluftleitung effektiv unterdrückt, so dass die Gefahr von Fehl-Erfassungen eines Endes der Expirationsphase durch das Beatmungsgerät geringer wird.

In der strömungsgünstigen Ausgestaltung des Ventilplenums, insbesondere derart, das entlang des im Ventilplenum ausgebildeten Fluidströmungswegs vom Fluideinlass zum Fluidauslass nur abgerundete Konturen gebildet sind, wird ein eigenständig schützenswerter Gegenstand der vorliegenden Erfindung gesehen. Die Anmelderin behält sich vor, darauf einen eigenständigen Schutz zu beanspruchen, unabhängig vom Vorhandensein einer oder mehrerer um den Fluideinlass herum ausgebildeter Versteifungsrippen. Zur strömungsgünstigen Ausgestaltung kann zudem auch die bereits oben angesprochene Ausgestaltung eines Ventilbodens, der sich in einem Bereich um den Fluideinlass herum in axialer Richtung auswölbt, beitragen.

In bestimmten Ausführungsformen kann der Fluidströmungsweg einen sich zum Fluidauslass hin erweiternden Querschnitt aufweisen. In dieser Ausgestaltung ist der Fluidströmungsweg besonders gut an den zum Fluidauslass hin immer größer werdenden Volumenstrom des während der Expiration durch das in dieser Phase geöffnete Expirationsventil strömenden Fluids (der ausgeatmeten Luft) angepasst. Je größer der Volumenstrom, desto größer der im Fluidströmungsweg bereitstehende Querschnitt. Insbesondere verändern sich auf jeweils gleich große Winkelbereiche bezogene Teilvolumina des Fluidströmungswegs entlang eines Umfangs des Fluideinlasses, und zwar derart, dass diese Teilvolumina in Richtung zum Fluidauslass hin immer größer werden. Das vermindert die Ausbildung von Turbulenz und Störungen in der Fluidströmung und schafft die Möglichkeit, dass das Expirationsventil sehr viel zuverlässiger, aber gleichzeitig auch empfindlicher, auf Veränderungen der Strömung ausgeatmeter Luft in dem Atemwegen regieren kann. Druck-Rückschläge, die bei herkömmlichen Expirationsventilen häufig zu den genannten Autotrigger-Fehlsteuerungen führten, treten sehr viel weniger in Erscheinung.

Der Querschnitt des Fluidströmungswegs soll als Fläche des für die Fluidströmungzur Verfügung stehenden Raum in quer, insbesondere orthogonal, zur hauptsächlichen Richtung der Fluidströmung zwischen Fluideinlass und Fluidauslass bei geöffnetem Expirationsventil stehenden Schnittflächen verstanden werden.

Insbesondere kann vorgesehen sein, dass die axiale Auswölbung eine sich um den Umfang des Fluideinlasses herum verändernde Tiefe hat. Die Tiefe der Auswölbung lässt sich dabei als ein in axialer Richtung gemessenen Abstand zwischen dem Ventilboden am Rand des Fluideinlasses und dem Ventilboden an der tiefsten Stelle der Auswölbung entlang eines Schnitts vom Rand des Fluideinlasses zum äußeren Rand des Ventilbodens bestimmen. Insbesondere soll die Tiefe der Auswölbung in Umfangsrichtung zu dem Fluidauslass hin zunehmen. Damit verändern sich auf jeweils gleich große Winkelbereiche bezogene Teilvolumina der Auswölbung entlang des Umfangs des Fluideinlasses, und zwar derart, dass diese Teilvolumina in Richtung zum Fluidauslass hin immer größer werden. In bestimmten Ausführungsformen kann die Tiefe der Auswölbung in Umfangsrichtung auf einer dem Fluidauslass gegenüber liegenden Seite am kleinsten sein und in Umfangsrichtung auf der zum Fluidauslass hin gelegenen Seite am größten sein.

Wie bereits angesprochen, kann der Fluideinlass insbesondere in einem zentralen Bereich des Ventilplenums angeordnet sein. Der Fluidauslass kann bezogen auf den Fluideinlass weiter außen angeordnet sein, insbesondere in radialer Richtung weiter außen, insbesondere an einem äußeren Rand des Ventilplenums. Der Querschnitt des Fluidströmungswegs zwischen dem Fluideinlass und dem Fluidauslass ergibt sich bei solcher Ausgestaltung jeweils als die Querschnittsfläche des Ventilplenums in einem jeweiligen von der Fluideinlassachse ausgehenden und zum äußeren Rand des Ventilplenums reichenden radialen Schnitt.

In bestimmten Ausführungsformen kann der Ventilkörper einen Fluideinlassteil aufweisen, in dem der Fluideinlass ausgebildet ist. Insbesondere kann der Fluideinlassteil integral mit dem Ventilkörper ausgebildet sein, also ebenfalls als Spritzgussteil für den Fall, dass der Ventilkörper ein Spritzgussteil ist. Der Fluideinlassteil kann sich insbesondere von dem Ventilboden weg erstrecken, und zwar insbesondere von einer Unterseite des Ventilbodens weg. Der Fluideinlassteil kann sich beispielsweise orthogonal zu dem Ventilboden erstrecken. Die Fluideinlassachse bezeichnet eine gedachte Linie in Längsrichtung des Fluideinlasssteils, die bezüglich des Fluideinlassteils zentral liegt. Bei orthogonal zum Fluideinlass stehender Fluideintrittsfläche steht die Fluideinlassachse orthogonal zu der durch den Fluideinlass definierten Fluideintrittsfläche und liegt bezüglich der Fluideintrittsfläche zentral. In bestimmten Ausführungsformen kann der Fluideinlasssteil einen ersten zapfenartigen Ansatz ausbilden, der zum Anschluss eines Atemluftschlauches ausgebildet sein kann. Beispielsweise kann der erste zapfenartige Ansatz zylindrisch oder konisch geformt sein und insbesondere als ein zylindrischer oder konischer Rohrstutzen ausgebildet sein, in welchem der Fluideinlass ausgebildet ist. Der Fluideinlass kann insbesondere einen kreisförmigen Querschnitt aufweisen.

Weiterhin kann insbesondere der Ventilkörper einen Fluidauslassteil bilden, in dem der Fluidauslass ausgebildet ist. Der Fluidauslassteil kann insbesondere einen zweiten zapfenartigen Ansatz ausbilden. Der Fluidauslassteil kann insbesondere integral mit dem Ventilkörper ausgebildet sein, beispielsweise ebenfalls als Spritzgussteil. Der zweite zapfenartige Ansatz kann insbesondere zylindrisch oder konisch geformt sein. Falls gewünscht, kann der zweite zapfenartige Ansatz beispielsweise als ein zylindrischer oder konischer Rohrstutzen ausgebildet sein, in welchem der Fluidauslass ausgebildet ist. Oft wird der zweite Ansatz aber unmittelbar in die Umgebung münden. Der Fluidauslass kann insbesondere einen kreisförmigen Querschnitt aufweisen.

Der sich auswölbende Bereich des Ventilbodens, welcher den Fluidströmungsweg zwischen Fluideinlass und Fluidauslass definiert, kann in den Fluidauslassteil übergehen. Der sich auswölbende Bereich des Ventilbodens kann insbesondere in eine Seitenwand des Fluidauslassteils übergehen. Insbesondere hat am Übergang zum Fluidauslassteil die durch den sich auswölbenden Ventilboden definierte Rinne eine maximale Tiefe.

Wie der Fluideinlassteil kann sich der Fluidauslassteil insbesondere vom Ventilboden weg erstrecken, insbesondere von einer Unterseite des Ventilbodens weg. Der Fluideinlassteil kann sich orthogonal zu dem Ventilboden erstrecken.

Weiterhin ist es günstig, wenn der Ventilkörper ferner eine Auflage aufweist, an deren Oberseite eine Ventilmembran mit einer an ihrem äußeren Rand ausgebildeten Anlagefläche auflegbar ist, wobei die die Auflage auf ihrer Oberseite uneben ausgebildet ist.

Wie bereits beschrieben, weist das Expirationsventil ferner eine dem Fluideinlass zugeordnete Ventilmembran auf, die mindestens zwischen einer den Fluideinlass verschließenden Stellung und einer den Fluideinlass öffnenden Stellung verlagerbar ist. Die Ventilmembran kann ohne eigenen Antrieb ausgebildet sein, so dass sie lediglich durch Druckunterschiede zwischen ihrer dem Fluideinlass zugewandten Seite und ihrer vom Fluideinlass abgewandten Seite zwischen Verschlussstellung und Freigabestellung verlagerbar ist. Zusätzlich oder alternativ kann aber auch ein Aktor vorgesehen sein, der die Ventilmembran zwischen Verschlussstellung und Freigabestellung hin und her verlagert. Dieser Aktor wird vorzugsweise vom Beatmungsgerät angesteuert. Diese Ventilmembran liegt mit der an ihrem äußeren Rand ausgebildeten Anlagefläche auf der der Oberseite der Auflage auf. Das Expirationsventil weist insbesondere ferner ein Spannelement auf, beispielsweise einen Spannring, welcher mit dem Ventilkörper derart zusammenwirkt, dass das Spannelement in Spanneingriff oder Klemmeingriff mit der Ventilmembran gelangt. Durch diesen Spanneingriff, der insbesondere am äußeren Umfang der Ventilmembran durch Klemmen mit dem Spannelement erfolgt, wird die Ventilmembran am Ventilkörper fixiert. Die Ventilmembran kann dabei durch das Spannelement so am Ventilkörper fixiert sein, dass sie bei Überdruck auf ihrer dem Fluideinlass zugeordneten Seite gegenüber ihrer dem Ventilplenum zugeordneten Seite in eine den Fluideinlass gegenüber dem Ventilplenum freigebende Stellung bringbar ist. Solange das nicht der Fall ist, insbesondere bei Druckgleichheit oder Unterdruck auf ihrer dem Fluideinlass zugeordneten Seite gegenüber ihrer dem Ventilplenum zugeordneten Seite verbleibt die Ventilmembran in einer den Fluideinlass gegenüber dem Ventilplenum verschließenden Stellung.

Die Erfindung ist durch die beigefügten Ansprüche definiert.

Die vorliegende Erfindung betrifft darüber hinaus ein Beatmungsgerät, welches ein an eine Expirationsluftleitung anschließbares Expirationsventil der vorangehend beschriebenen Art aufweist.

Die Erfindung wird nachfolgend anhand eines Ausführungsbeispiels unter Bezugnahme auf die Figuren näher erläutert. Es zeigt:
Fig. 1: Eine Beatmungsmaschine zur maschinellen Beatmung von Patienten, mit Steuereinheit, Monitor, sowie Schläuchen für zugeführte Beatmungsluft und für ausgeatmete Luft;
Fig. 2: Ein Expirationsventil gemäß einer Ausführungsform in Schnittansicht;
Fig. 3: Den Ventilkörper des Expirationsventils aus Fig. 2 in einer perspektivischen Ansicht von unten; und
Fig. 4: Den Ventilkörper des Expirationsventils aus Fig. 2 in einer perspektivischen Ansicht von oben.

In allen Figuren werden für jeweils gleiche oder funktionsgleiche Bauteile dieselben Bezugszeichen verwendet. Eine detaillierte Beschreibung solcher Bauteile erfolgt jeweils nur für die erste Figur, in der das jeweilige Bezugszeichen erscheint. Es versteht sich, dass dieselbe Beschreibung auch für alle anderen Figuren gilt, in denen das mit demselben Bezugszeichen versehene Bauteil zu sehen ist, es sei dann, dass ausdrücklich anderes vermerkt ist.

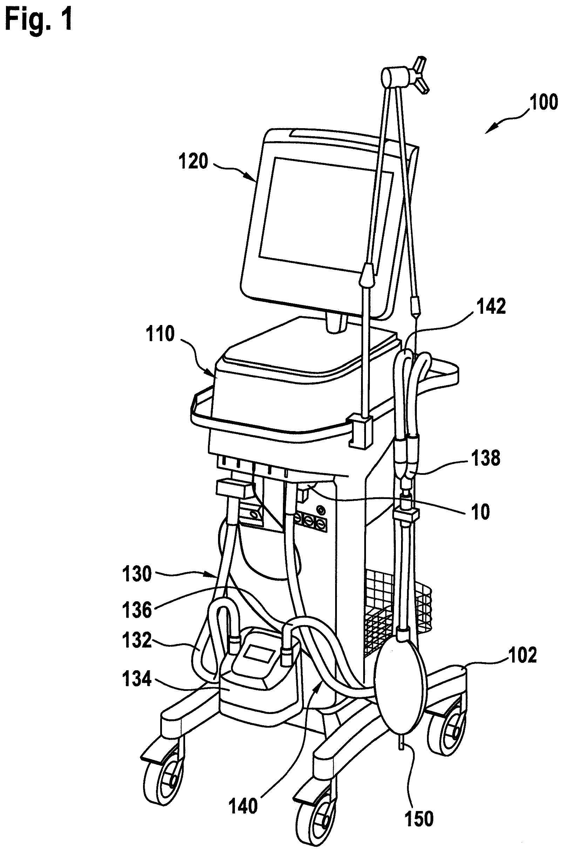

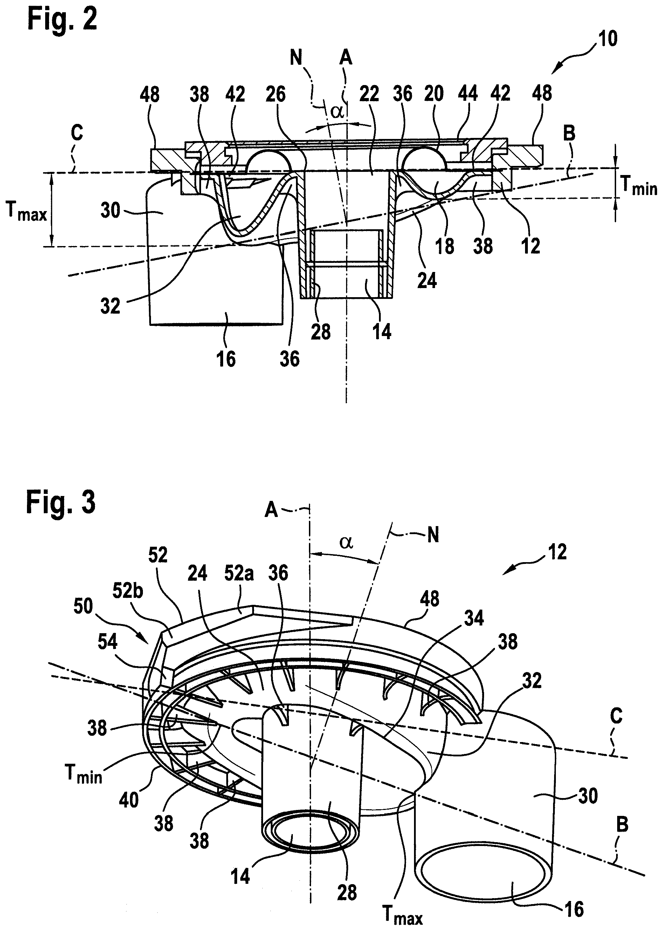

Fig. 1 zeigt eine Beatmungsmaschine 100 zur maschinellen Beatmung von Patienten. Die Beatmungsmaschine 100 umfasst ein auf Rollen fahrbares Gestell 102 mit Steuereinheit 110 und Monitor 120 sowie eine Leitung 130 für von der Maschine zugeführte Beatmungsluft und eine Leitung 140 für ausgeatmete Luft. Die Leitung 130 für zugeführte Beatmungsluft umfasst einen von der Steuereinheit 110 ausgehenden ersten Beatmungsluftschlauch 132, der zu einer Befeuchtungseinheit 134 führt, wo die Beatmungsluft durch ein Wasserreservoir geleitet wird. Von der Befeuchtungseinheit 134 führt ein zweiter Beatmungsluftschlauch 136 zu einem T-Stück 138. Von dem T-Stück gelangt die von der Beatmungsmaschine 100 während einer Inspirationsphase des Beatmungszyklus bereitgestellte Beatmungsluft über eine Leitung 150 zum Patienten. Über die Leitung 150 gelangt auch die vom Patienten während einer Expirationsphase des Beatmungszyklus ausgeatmete Luft zurück zur Beatmungsmaschine 100. Hierzu zweigt von dem T-Stück 138 ein weiterer Atemluftschlauch 142 ab, der zu der Leitung 140 für ausgeatmete Luft gehört. Der Atemluftschlauch 142 führt zu einem allgemein mit 10 bezeichneten Expirationsventil, welches nachfolgend unter Bezugnahme auf die Figuren 2 bis 4 noch näher erläutert werden wird.

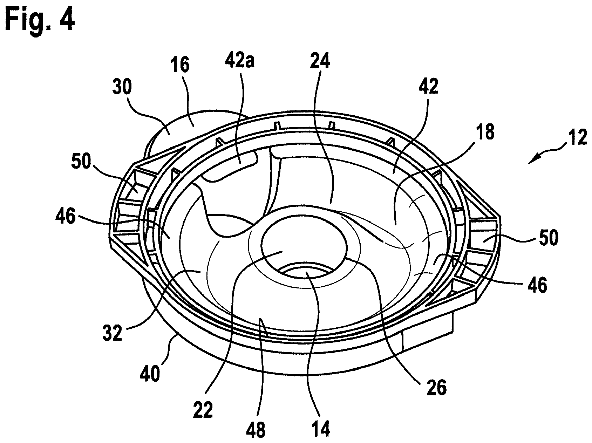

Fig. 2 zeigt in Schnittansicht das in Fig. 1 eingesetzte Expirationsventil 10 gemäß einer Ausführungsform. Das Expirationsventil 10 umfasst einen Ventilkörper 12, in dem ein Fluideinlass 14 und ein Fluidauslass 16 für Atemluft, die von einem Patienten ausgeatmet wird, ausgebildet sind. Die Fig. 3 und 4 zeigen perspektivische Ansichten dieses Ventilkörpers 12, und zwar zeigt Fig. 3 eine Ansicht von unten in etwa aus Richtung des mit in Fig. 4 mit 12 bezeichneten Pfeils, und zeigt Fig. 4 eine Ansicht von oben in etwa aus Richtung des in Fig. 3 mit 12 bezeichneten Pfeils. In Fig. 2 ist das Expirationsventil 10 vor dem Einbau in eine Beatmungsmaschine 100 gezeigt, so dass der Atemluftschlauch 142 noch nicht angeschlossen ist. Im betriebsgemäßen Zustand, d.h. nach Einbau des Expirationsventils 10 in eine Beatmungsmaschine 100 gemäß Fig. 1, ist der Atemluftschlauch 142 an dem Fluideinlass 14 angeschlossen. Somit gelangt vom Patienten ausgeatmete Luft über den Fluideinlass 14 zum Expirationsventil 10. Nach Durchgang durch das Expirationsventil 10 gelangt die ausgeatmete Luft über den Fluidauslass 16 in eine Umgebung des Beatmungsgeräts 100.

Das Expirationsventil 10 umfasst ein sogenanntes Ventilplenum 18. Das Ventilplenum 18 bildet einen Hohlraum, der über den Fluideinlass 14 mit Fluid (ausgeatmete Atemluft) beaufschlagbar ist und aus dem über den Fluidauslass 16 Fluid (ausgeatmete Atemluft) ausströmen kann. In dem Ventilplenum 18 ist somit durch den Ventilkörper 12 und eine Ventilmembran 20 ein Fluidströmungsweg zwischen dem Fluideinlass 14 und dem Fluidauslass 16 gebildet. Der Fluideinlass 14 ist so ausgebildet, dass Atemluft beim Ausatmen, d.h. während einer Expirationsphase des Beatmungszyklus, in das Ventilplenum 18 gelangen kann. Während einer Inspirationsphase des Beatmungszyklus, d.h. wenn dem Patienten über die Beatmungsleitung 130 maschinell Atemluft zugeführt wird, soll der Fluideinlass 14 verschlossen sein. Hierfür dient die dem Fluideinlass 14 zugeordnete Ventilmembran 20. Die Ventilmembran 20 ist mindestens zwischen einer den Fluideinlass 14 verschließenden Stellung und einer den Fluideinlass 14 öffnenden Stellung verlagerbar. Die Ventilmembran 20 ist insbesondere so ausgebildet, dass ein zentraler Bereich der Ventilmembran 20 eine vom Fluideinlass 14 gebildete Fluid eintrittsöffnung 22 verschließend am Fluideinlass 14 anliegt, solange der Druck auf der dem Fluideinlass 14 zugeordneten Seite der Ventilmembran 20 nicht um einen vorbestimmten Druck größer ist als auf der auf der vom Fluideinlass 14 abgewandten Seite der Ventilmembran 20 herrschende Druck (das ist normalerweise der Umgebungsdruck). Die Ventilmembran 20 befindet sich dann in einer den Fluideinlass 14 bzw. die Leitung 140 gegenüber dem Ventilplenum 18 verschließenden Stellung. Diese Stellung nimmt die Ventilmembran 20 während der Inspirationsphasen ein. Bei genügend großem Überdruck auf ihrer dem Fluideinlass 14 bzw. der Leitung 140 für ausgeatmete Atemluft zugeordneten Seite gegenüber ihrer vom Fluideinlass 14 bzw. der Leitung 140 für ausgeatmete Atemluft abgewandten Seite bewegt sich die Ventilmembran 20 in eine den Fluideinlass 14 gegenüber dem Ventilplenum 18 freigebende Stellung, so dass Atemluft aus der Leitung 140 für ausgeatmete Atemluft in das Ventilplenum 18 gelangen kann. Dies geschieht üblicherweise während der Expirationsphasen. Die Ventilmembran 20 ist aus einem Kunststoffmaterial hergestellt, insbesondere aus einem Kunststoffmaterial mit elastischen Eigenschaften, beispielsweise einem Silikonkunststoff. Dabei kann die Ventilmembran 20 so eingebaut sein, dass sie ohne Beaufschlagung durch einen Überdruck auf ihrer der Leitung 140 für ausgeatmete Atemluft zugewandten Seite unter Vorspannung an einem die Fluideintrittsöffnung 22 umgebenden Rand 26 des Fluideinlasses 14 anliegt. Bei Beaufschlagung mit einem genügend großen Überdruck auf ihrer der Leitung 140 für ausgeatmete Atemluft zugewandten Seite löst sich die Ventilmembran 20 von dem die Fluideintrittsöffnung 22 umgebenden Rand 26 des Fluideinlasses 14, so dass ausgeatmete Atemluft in das Ventilplenum 18 gelangen kann. Zusätzlich oder alternativ kann aber auch ein Aktor vorgesehen sein, der die Ventilmembran 20 zwischen Verschlussstellung und Freigabestellung hin und her verlagert. Dieser Aktor wird vorzugsweise vom Beatmungsgerät angesteuert und greift beispielsweise in dem zentralen Bereich der Ventilmembran 20 auf der von der Fluid eintrittsöffnung 22 abgewandten Seite der Ventilmembran 20 an. Die jeweilige Stellung der Ventilmembran 20 bestimmt dabei das Volumen des Ventilplenums 18 und den zwischen Ventilmembran 20 und Ventilkörper 12 gebildeten Fluidströmungsweg.

Der Ventilkörper 12 weist einen Ventilboden 24 auf, der sich auf einer Unterseite des Ventilplenums 18 von dem Fluideinlass 14 ausgehend nach außen bis zu einem äußeren Rand des Ventilplenums bzw. Ventilbodens erstreckt. Der Ventilboden 24 definiert zusammen mit der Ventilmembran 20 den im Ventilplenum 18 gebildeten Fluidströmungsweg zwischen dem Fluideinlass 14 und dem Fluidauslass 16. Insbesondere bildet der Ventilkörper 12 in bestimmten Ausführungsformen ein topfförmiges Ventilplenum 18, welches wenigstens zum Teil an seiner Unterseite von dem Ventilboden 24 begrenzt ist und in das der Fluideinlass 14 mündet. Der Ventilboden 24 kann sich dabei insbesondere tellerartig zwischen dem Fluideinlass 14 und einem äußeren Rand des Ventilkörpers 12 (insbesondere des Ventilplenums 18) erstrecken, wobei der äußere Rand des Ventilbodens 24 zugleich den äußeren Rand des Ventilkörpers 12 bildet. Der Fluideinlass 14 ist dann insbesondere in einem zentralen Teil des Ventilbodens 24 ausgebildet. Der Fluid einlass 14 kann insbesondere einen kreisförmigen Querschnitt aufweisen mit ebenfalls kreisförmiger Fluideintrittsfläche 22. Ein die Fluideintrittsfläche 22 definierender Rand 26 des Fluideinlasses 14 bildet damit einen inneren Rand des Ventilbodens 24, der sich ausgehend von dem Rand 26 an der Unterseite des Ventilplenums 18 bis zu einem äußeren Rand des Ventilkörpers 12 erstreckt. Der äußere Rand des Ventilkörpers 12 befindet sich in radialer Richtung am weitesten vom Fluideinlass 14 weg. Damit steht der Rand 26 nicht - oder jedenfalls nicht abrupt oder scharfkantig - vom Ventilboden 24 hervor, sondern vielmehr beginnt der Ventilboden 24 unmittelbar am Rand 26 der Fluideintrittsöffnung 22 und erstreckt sich in einer sanft geschwungenen Kontur von diesem Rand 26 aus nach außen. Durch diese Formgebung werden scharfe Kanten vermieden, die bei Umströmung mit Luft zu Strömungsabrissen und damit verbundener Geräuschentwicklung führen könnten.

Der Ventilboden 24 hat eine tellerartige Gestalt. Damit soll zum Ausdruck gebracht werden dass der Ventilboden 24 sich im Wesentlichen flach oder allenfalls flach konisch ausgebildet zwischen seinem inneren Rand 26 und dem äußeren Rand erstreckt. Etwaige Vertiefungen im Ventilboden 24, beispielsweise eine nachfolgend noch näher zu beschreibende Rinne, sind so ausgebildet, dass sich eine insgesamt abgerundete und strömungsangepasste Kontur ergibt mit sanft ineinander übergehenden unterschiedlichen Krümmungen.

Während der Fluideinlass 14 in einem zentralen Bereich des Ventilplenums 18 angeordnet ist, liegt der Fluidauslass 16 bezogen auf den Fluideinlass 14 radial weiter außen. Insbesondere ist der Fluidauslass 16 an einem äußeren Rand des Ventilplenums 18 angeordnet. An der Stelle des Ventilauslasses 16 ist im Ventilkörper 12, insbesondere in einer äußeren Wand des Ventilbodens 24 eine Austrittsöffnung ausgebildet durch die Atemluft aus dem Ventilplenum 18 zum Fluidauslass 16 strömen kann. Auch die Austrittsöffnung ist mit sanft geschwungenen und damit strömungsgünstig ausgebildeten Kanten versehen, so dass ein Entlangströmen von Atemluft an scharfen Kanten beim Austritt aus dem Ventilplenum 18 vermieden wird.

Der Ventilkörper 12 ist aus Kunststoffmaterial hergestellt, beispielsweise aus Polyethylen, Polypropylen oder einem anderen thermoplastischen Kunststoffmaterial. Der Ventilkörper 12 ist ein Spritzgussteil, d.h. er ist in einem Spritzgussverfahren hergestellt. Insbesondere kann der Ventilkörper 12 so ausgebildet sein, dass er in einem einzigen Spritzgussprozess herstellbar ist, insbesondere als ein Einkomponenten-Spritzgussteil.

Der Ventilkörper 12 weist einen Fluideinlassteil 28 auf, in dem der Fluideinlass 14 ausgebildet ist. Der Fluideinlassteil 28 ist insbesondere integral mit dem Ventilkörper 12 ausgebildet. Der Fluideinlassteil 28 erstreckt sich von dem Ventilboden 24 weg, und zwar in Richtung von einer Unterseite des Ventilbodens 24 weg im Wesentlichen entlang einer Achsrichtung A des Fluideinlasses 14. Zudem erstreckt sich der Fluideinlassteil 28 quer zu dem Ventilboden 24, d.h. er steht von einer Unterseite des Ventilbodens 24 in Achsrichtung A des Fluideinlasses 14 vor. Die Achsrichtung A bezeichnet eine Fluideinlassachse, d.h. eine gedachte Linie in Längsrichtung des Fluideinlasssteils 28, die bezüglich des Fluideinlasses 14 zentral liegt. Bei orthogonal zum Fluideinlass 14 stehender Fluideintrittsfläche 22 steht die Fluideinlassachse A orthogonal zu der durch den Fluideinlass 14 definierten Fluideintrittsfläche 22 und liegt bezüglich der Fluideintrittsfläche 22 zentral.

Der Fluideinlasssteil 28 bildet einen ersten zapfenartigen Ansatz aus, der insbesondere zum Anschluss eines Atemluftschlauches, beispielsweise des in Fig. 1 zu sehenden Atemluftschlauches 142, ausgebildet ist. In der in Fig. 2 gezeigten Variante ist der erste zapfenartige Ansatz insbesondere zylindrisch geformt. In anderen Varianten könnte der zapfenartige Ansatz auch sich zu seinem freien Ende hin leicht verjüngend ausgebildet sein, beispielsweise leicht konisch zulaufend. Der erste zapfenartige Ansatz kann beispielsweise als ein Rohrstutzen ausgebildet sein, in welchem der Fluideinlass 14 ausgebildet ist.

Der Ventilkörper 12 bildet zudem einen Fluidauslassteil 30, in dem der Fluidauslass 16 ausgebildet ist. Der Fluidauslassteil 30 kann insbesondere einen zweiten zapfenartigen Ansatz ausbilden, der zylindrisch oder sich verjüngend (insbesondere konisch) geformt sein kann. Der zweite zapfenartige Ansatz kann beispielsweise als ein zylindrischer Rohrstutzen ausgebildet sein, in welchem der Fluidauslass 16 ausgebildet ist. Auch der Fluidauslassteil 30 kann insbesondere integral mit dem Ventilkörper 12 ausgebildet sein. Der Fluidauslass 16 kann insbesondere einen kreisförmigen Querschnitt aufweisen. Er braucht nicht unbedingt an eine weitere Leitung angeschlossen zu sein, sondern kann direkt in die Umgebung münden, so dass ausgeatmete Luft über den Fluidauslass 16 in die Umgebung abgegeben wird. An seiner radial inneren Seite ist der Fluidauslassteil 30 über die bereits angesprochene Austrittsöffnung mit dem Ventilplenum 18 verbunden, so dass in das Expirationsventil 10 eingeströmtes Fluid über die Austrittsöffnung in den Fluidauslassteil 30 gelangt.

Ähnlich wie der Fluideinlassteil 28 kann sich der Fluidauslassteil 30 insbesondere vom Ventilboden 24 weg erstrecken, insbesondere von einer Unterseite des Ventilbodens 24 weg. Der Fluidauslassteil 30 kann sich orthogonal zu dem Ventilboden 24 erstrecken und/oder in einer Richtung parallel zu dem Fluideinlassteil 28 erstrecken, wie das in den Fig. 2 bis 4 gut zu sehen ist.

Der Fluidströmungsweg in dem Ventilplenum 18 besitzt einen quer zur Fluidströmung zwischen Fluideinlass 14 und Fluidauslass 16 gemessenen Querschnitt, der sich von dem Fluideinlass 14 zum Fluidauslass 16 hin erweitert. Dies wird erreicht durch eine entsprechende Ausbildung und Anordnung des Ventilkörpers 12, insbesondere des Ventilbodens 24, und der Ventilmembran 20. Der Ventilboden 24 bildet vor allem eine bodenseitige Begrenzung des Fluidströmungswegs. Die Ventilmembran 20 bildet vor allem eine Begrenzung des Fluidströmungswegs auf dessen Oberseite. Während durchaus eine Ventilmembran 20 verwendet werden kann, die herkömmlichen Ventilmembranen ähnlich ist, ist der Ventilboden 24 so ausgestaltet, dass sich der Querschnitt des Fluidströmungswegs zum Ventilauslass 16 hin vergrößert. Insbesondere ist der Ventilboden 24 so ausgebildet dass sich sein Abstand zur Ventilmembran 20 vom Fluideinlass 14 her zum Fluideinlass 16 hin vergrößert. Dies kann in einer oder mehreren Stufen geschehen, insbesondere jedoch kontiniuierlich von dem Fluideinlass 14 zu dem Fluidauslass 16 hin. Bei der in Fig. 2 bis 4 zu sehenden Geometrie des Expirationsventils 10 mit radial innen und zentral angeordnetem Fluideinlass 14 und radial außen angeordnetem Fluidauslass 16 bietet es sich beispielsweise an, dass der Fluidströmungsweg so ausgestaltet ist, dass sich auf jeweils gleich große Winkelbereiche um den Fluideinlass 14 herum bezogene Teilvolumina des Fluidströmungswegs entlang des Umfangs des Fluideinlasses 14 verändern, und zwar derart, dass diese Teilvolumina in Richtung zum Fluidauslass 16 hin immer größer werden. Ein solcher Effekt lässt sich beispielsweise erreichen, wenn eine Ebene, entlang der sich der Ventilboden 24 erstreckt, in Bezug auf eine Ebene, entlang der sich die Ventilmembran 20 erstreckt, geneigt ist, und zwar derart, dass diese beiden Ebenen zum Fluidauslass 16 hin auseinander laufen. Während sich beispielsweise die Ventilmembran 20 in aller Regel in einer Ebene orthogonal zu der Fluideinlassachse A erstrecken wird (in Fig. 2 und 3 ist diese Ebene sehr schematisch durch eine mit C bezeichnete gestrichelte Linie angedeutet), kann in bestimmten Ausführungsformen der Ventilboden 24 sich entlang einer Ebene erstrecken, die gegenüber der Fluideinlassachse A in einem von 90 Grad verschiedenen Winkel geneigt ist. Die Ebene, entlang derer sich der Ventilboden 24 erstreckt, soll dabei die Ebene bezeichnen, die der mittleren Erstreckung des Ventilbodens 24 folgt, wobei von etwaigen einzelnen (etwa dellenförmigen) Erhebungen bzw. Vertiefungen des Ventilbodens 24 abzusehen ist. Besitzt allerdings der Ventilboden 24 eine durchgehend ausgebildete Vertiefung, die den Querschnitt des Fluidströmungswegs zwischen dem Fluideinlass 14 und dem Fluidauslass 16 bestimmt, wie dies nachfolgend noch beschrieben wird, so wird die Ebene, entlang der sich der Ventilboden 24 erstreckt, im Allgemeinen durch den Verlauf dieser Vertiefung (d.h. den Verlauf der tiefsten Stellen in den einzelnen entlang des Fluidströmungswegs aufeinander folgenden Querschnitten) bestimmt werden. In Fig. 2 und 3 ist die Lage dieser Ebene sehr schematisch durch die Linie B angedeutet. Man sieht, dass diese Ebene nicht parallel zu der Ebene ist, entlang der die Ventilmembran 20 verläuft (diese Ebene verläuft orthogonal zu der Fluideinlassachse A), sondern einen von 90 Grad verschiedenen Winkel zu der Fluideinlassachse A bildet. Das impliziert, dass der Normalenvektor N dieser Ebene nicht parallel zu der Fluideinlassachse A ist, sondern einen Winkel α zu dieser bildet. Im Normalfall wird der Normalenvektor N dabei gegenüber der Fluideinlassachse A zu dem dem Fluidauslass 16 hin geneigt sein, wie das auch in Fig. 2 und 3 angedeutet ist.

Die Vergrößerung des Querschnitts des Fluidströmungswegs zum Fluidauslass 16 hin trägt dem Umstand Rechnung, dass dass der Volumenstrom an zum Auslass strömender Atemluft immer mehr zunimmt je näher der Fluidströmungsweg dem Fluidauslass liegt. Das für den Volumentstrom zur Verfügung stehende Volumen im Ventilplenum 18 ist auf diese Weise an den Volumenstrom angepasst. Man vermeidet durch diese Anpassung des für den Fluidstrom zur Verfügung stehenden Volumens Staueffekte mit daraus resultierender Verwirbelung der auszuatmenden Atemluft.

In Fig. 2 bis 4 ist zudem gut zu sehen, dass der Ventilboden 24 sich in einem Bereich um den Fluideinlass 14 herum in axialer Richtung auswölbt. Mit "in axialer Richtung auswölben" soll hierbei gemeint sein, dass der Ventilboden 24 in wenigstens einem die Fluideinlassachse A enthaltenden Schnitt einen konvexen Verlauf hat, und zwar in einem zwischen dem Rand des Fluideinlasses 14 und dem äußeren Rand des Ventilbodens 24 liegenden Bereich. In den gezeigten Ausführungsformen kann dies ein in radialer Richtung verlaufender Schnitt sein. In Fig. 2 ist dieser konvexe Verlauf für den Schnitt durch die tiefste Stelle der so gebildeten Auswölbung 32 beim Fluidauslass 16 auf der linken Seite in Fig. 2 und für den Schnitt durch die Stelle mit geringster Tiefe der Auswölbung 32 auf der rechten Seite in Fig. 2 angedeutet. Konvex bedeutet, dass in einem solchen Schnitt eine zwei Punkte auf dem Ventilboden 24 verbindende Linie durch das Ventilplenum 18 verläuft. Der Ventilboden 24 bildet demgemäß im Bereich der so entstehenden Auswölbung 32 eine um den Fluideinlass 14 umlaufende Rinne aus, deren Boden sich zwischen dem Rand des Fluideinlasses 14 und dem äußeren Rand des Ventilbodens 24 in axialer Richtung auswölbt. Die Rinne definiert so den um den Fluideinlass 14 herum führenden Fluidströmungsweg. Der entlang des Fluidströmungswegs jeweils tiefste Punkt dieser Rinne (anders ausgedrückt: die Verbindung der in jeweiligen Schnitten zwischen Fluideinlass 14 und äußerem Rand des Ventilbodens 24 tiefsten Punkte) bildet so eine um den Fluideinlass 14 herum führende Linie, die in Fig. 3 mit 34 bezeichnet ist. Bei der in den Figuren gezeigten radialsymmetrischen Geometrie des Ventilbodens 24 mit Fluideinlass 14 in der Mitte ist diese Linie 34 wegen der noch näher zu erläuternden unterschiedlichen Tiefe der Rinne im Wesentlichen ellipsenförmig (die Projektion dieser Linie 34 auf eine zur Fluideinlassachse A orthogonale Ebene, wie die Ebene C, ist kreisförmig). Wie in Fig. 2 bis 4 gut zu sehen ist, bildet der sich auswölbende Bereich des Ventilbodens 24 (oder die so gebildete Rinne) zwei im Wesentlichen um den halben Umfang des Fluideinlasses 14 herum führende Fluidströmungswege aus, die schließlich an einer Seite (in Fig. 2 links vom Fluideinlass 14) in den Fluidauslass 16 münden. Dabei geht der sich auswölbende Bereich des Ventilbodens 24 mit seiner äußeren Seitenwand in den Fluidauslassteil 30 über. Wie bereits angesprochen, haben alle Kanten entlang dieses Fluidströmungswegs eine abgerundete Kontur, die Strömungsabrissen und/oder Verwirbelungen der Fluidströmung möglichst entgegenwirkt.

In den Figuren ist gut zu sehen, dass die axiale Auswölbung 32 des Ventilbodens 24 eine sich um den Umfang des Fluideinlasses 14 herum verändernde Tiefe hat. Die Tiefe ist dabei zu messen als ein in Richtung der Fluideinlassachse A gemessener Abstand zwischen dem Ventilboden 24 am Rand 26 des Fluideinlasses 14 und dem Ventilboden 24 an der tiefsten Stelle der Auswölbung 32 in einem den Fluideinlass 14 mit dem äußeren Rand des Ventilbodens 24 verbindenden Schnitt (bei radialsymmetrischer Geometrie des Ventilbodens 24, wie in Figuren gezeigt, ist das ein radialer Schnitt durch den Ventilboden 24). In Fig. 2 und 3 ist die jeweils größte Tiefe Tmax an der Stelle des Fluidauslasses 16 und die jeweils kleinste Tiefe Tmin an der dem Fluidauslass 16 gegenüber liegenden Seite angedeutet.

Auf diese Weise verändern sich auf jeweils gleich große Winkelbereiche bezogene Teilvolumina des Fluidströmungswegs entlang des Umfangs des Fluideinlasses 14, und zwar derart, dass diese Teilvolumina in Richtung zum Fluidauslass 16 hin immer größer werden. Es hat sich gezeigt, dass diese Maßnahme ganz erheblich zur Reduzierung von strömungsbedingten Geräuschen beim Abgeben von Atemluft während Expirationsphasen beiträgt, weil das für die Fluidströmung zur Verfügung stehende Volumen dem Volumenstrom des strömenden Fluids angepasst ist.

Zudem ist in den Figuren gut zu sehen, dass der Ventilboden 24 ausgehend von einer Innenseite des Fluideinlasses 14 am Rand der Fluideintrittsfläche 22 auf seiner dem Ventilplenum 18 zugewandten Seite eine abgerundete Kontur aufweist. Dies ist insbesondere in Fig. 4 gut zu erkennen. Der Fluidstrom strömt nach Passieren der Fluideintrittsfläche 22 entlang der abgerundeten inneren Ränder der durch die Auswölbung 32 gebildeten Rinne zu deren tiefster Stelle, um dann entlang des durch die Rinne gebildeten Fluidströmungswegs - etwa der Linie 34 folgend - um den Fluideinlass 14 herum dem Fluidauslass 16 entgegen zu strömen. Hierbei passiert die Fluidströmung keinerlei scharfe Kanten, die zu Strömungsabrissen oder Verwirbelungen mit damit verursachter Geräuschentwicklung führen würden.

Der Ventilkörper 12 weist außerdem um den Fluideinlass 14 herum ausgebildete Versteifungsrippen 36, 38 auf. In Fig. 2 und 3 sind beispielhaft einige dieser Versteifungsrippen mit den Bezugszeichen 36 und 38 bezeichnet. Die Versteifungsrippen 36, 38 versteifen den Ventilboden 24, insbesondere den durch den Ventilboden 24 gebildeten Mantel im sich auswölbenden Bereich 32. Verwindungen des Ventilkörpers 12 werden durch diese zusätzliche Versteifung effektiv unterdrückt. Damit tragen die Versteifungsrippen 36, 38 ebenfalls dazu bei, Geräuschentwicklung zu verringern, weil sie Schwingungen des Ventilbodens 24 unter dem Einfluss von Fluidströmung im Ventilplenum 18 unterdrücken. Damit lassen sich aber auch ungewollte störende Schwingungen der Ventilmembran 20 effektiv unterdrücken. Dadurch sinkt die Gefahr für Oszillationen der Luftsäule im Leitungssystem, welche zu fälschlich ausgelösten Steuervorgängen (insbesondere sogenannte Autotrigger) führen können. Die Versteifungsrippen 36, 38 sind integral mit dem Ventilkörper 12 ausgebildet. Integral ist dabei so verstehen, dass die Versteifungsrippen 26 28 und der Ventilkörper 12 als ein einziges Bauteil hergestellt sind. In dem gezeigten Ausführungsbeispiel sind die Versteifungsrippen 36, 38 im selben Spritzgussprozess hergestellt wie der Ventilkörper 12.

Die Versteifungsrippen 36, 38 sind außerhalb des Fluideinlasses 14 angeordnet. Wie in den Figuren zu sehen ist, verlaufen sie bezogen auf den Rand 26 des Fluideinlasses 14 nach außen zum Rand des Ventilkörpers 12 hin. In dem gezeigten Ausführungsbeispiel verlaufen die Versteifungsrippen bezüglich der Fluideinlassachse A in radialer Richtung. Sie könnten aber auch eher spiralförmig von innen nach außen führen. Die Versteifungsrippen verbinden dabei den Rand 26 des Fluideinlasses 14 mit dem äußeren Rand des Ventilkörpers 12. Die Versteifungsrippen 36, 38 sind auf einer Unterseite des Ventilbodens 24 ausgebildet. Somit stören sie den auf der Innenseite des Ventilkörpers 12, d.h. an der Oberseite des Ventilbodens 24 ausgebildeten Fluidströmungsweg nicht. Wie in den Figuren zu sehen ist, umfassen die Versteifungsrippen erste Versteifungsrippen 36 und zweite Versteifungsrippen 38. Die ersten Versteifungsrippen 36 sind innere Versteifungsrippen, die zwischen dem Rand 26 des Fluideinlasses 14 und dem sich auswölbenden Bereich 32 des Ventilbodens 24 verlaufen. In dem gezeigten Ausführungsbeispiel verbinden die ersten Versteifungsrippen 36 den Rand 26 des Fluideinlasses 14 mit dem sich auswölbenden Bereich 32 des Ventilbodens 24. Die zweiten Versteifungsrippen 38 sind äußere Versteifungsrippen, die zwischen dem sich auswölbenden Bereich des Ventilbodens 32 und dem äußeren Rand des Ventilbodens 24 verlaufen. In dem gezeigten Ausführungsbeispiel verbinden die zweiten Versteifungsrippen 38 den sich auswölbenden Bereich 32 des Ventilbodens 24 mit dem äußeren Rand des Ventilbodens 24. Die zweiten Versteifungsrippen 38 enden in einem am äußeren Rand des Ventilbodens 24 ausgebildeten flanschartigen Ansatz 40. Der Ansatz 40 kann beispielsweise mindestens einen um den äußeren Rand des Ventilbodens 24 umlaufenden Absatz aufweisen, insbesondere mindestens zwei im Abstand zueinander angeordnete Absätze, wobei die zweiten Versteifungsrippen 38 in den Absatz, bzw. einen inneren der Absätze, übergehen. Der Absatz bzw. die Absätze kann beispielsweise einen ringartigen Flansch bilden, der um den äußeren Rand des Ventilbodens 24 umläuft und diesem Stabilität verleiht.

Wie in Fig. 2 gezeigt ist, weist das Expirationsventil 10 ferner die dem Fluideinlass 14 zugeordnete Ventilmembran 20 auf. Die Ventilmembran 20 liegt im in das Expirationsventil 10 eingebauten Zustand mit einer an ihrem äußeren Rand ausgebildeten Anlagefläche an der Oberseite einer Auflage 42 an, die an der Oberseite des Ventilkörpers 12 ausgebildet ist. Die Auflage 42 bildet einen um den äußeren Rand des Ventilkörpers 12 umlaufenden Vorsprung, der von dem äußeren Rand des Ventilkörpers 12 nach innen, zur Seite des Ventilplenums 18, vorspringt. Die Auflage 42 ist ebenfalls integral mit dem Ventilkörper 12 ausgebildet, beispielsweise als Spritzgussteil im selben Spritzgussprozess ausgebildet wie der Ventilkörper 12. Beim Zusammenbau des Expirationsventils 10 wird die Ventilmembran 20 mit ihrer äußeren Anlagefläche in Anlage gebracht mit der Oberseite der Auflage 42 des Ventilkörpers 12, so dass die Ventilmembran 20 mit einer an ihrem zentralen Teil ausgebildeten Anlagefläche auf dem Rand 26 des Fluideinlasses 14 aufliegt und mit der an ihrem äußeren Teil ausgebildeten Anlagefläche an der Auflage 42 aufliegt. Die Ventilmembran 20 bildet so eine Oberseite des im Ventilplenum 18 gebildeten Fluidströmungswegs. Die Ventilmembran 20 wird dann mittels eines in Fig. 2 gezeigten Spannrings 44 gegenüber dem Ventilkörper 12 eingespannt. Hierzu wird der Spannring 44 auf die Ventilmembran aufgesetzt und dann bajonettartig gegenüber dem Ventilkörper 12 verdreht. Durch die so entstehenden Spannkräfte verformt sich die Ventilmembran 20 an ihrem Umfang in gewissem Umfang.

Die Auflage 42 ist deswegen auf ihrer Oberseite uneben ausgebildet. Die so gebildeten in Fig 4 mit dem Bezugszeichen 46 angedeuteten Unebenheiten der Oberfläche der Auflage 42 kompensieren die beim Verspannen des Spannrings 44 gegenüber dem Ventilkörper 12 entstehende Verformung der Ventilmembran 20, so dass die Bildung von Falten oder Verwerfungen am Umfang der Ventilmembran 20 im eingespannten Zustand unterdrückt werden kann. Insbesondere sind an der Oberseite der Auflage 42 Unebenheiten 46 in Gestalt von entlang des Umfangs im Abstand voneinander angeordneten Auswölbungen oder Rippen ausgebildet. Diese Auswölbungen oder Rippen können einen radialen Verlauf haben. Noch besser ist es, wenn die Auswölbungen oder Rippen gewindeartig ausgebildet sind, also eine gewisse Steigung gegenüber der Ebene der aufliegenden Ventilmembran 20 aufweisen und/oder nicht nur in radialer Richtung verlaufen, sondern auch in Umfangsrichtung, beispielsweise einen spiralartigen Verlauf um den Rand des Ventilkörpers 12 herum besitzen. Um die erzeugten Spannkräfte zu begrenzen, kann es zudem günstig sein, die Steigung der durch die Auswölbungen oder Rippen gebildeten Gewindegänge gering zu halten und/oder den ansteigenden Bereich zu begrenzen, etwa durch einen Anschlag am Ende einer Auswölbung oder Rippe und/oder durch Übergang in einen parallel zur Ebene der Ventilmembran 20 verlaufenden Bereich am Ende der Auswölbung oder Rippe. Die Unebenheiten, insbesondere in Gestalt von Auswölbungen und/oder Rippen, können einteilig mit der Auflage 42 ausgebildet sein.

Die Auflage 42 kann um den Umfang des äußeren Randes des Ventilkörpers umlaufend ausgebildet sein (wie etwa in dem mit 42 bezeichneten Bereich in Fig. 4) oder mit Unterbrechungen ausgebildet sein, so dass sich einzelnen Auflagebereiche ergeben, die entlang des Umfangs des äußeren Randes des Ventilkörpers 12 an aufeinander folgen. In dem Ausführungsbeispiel gem. Fig. 4 ist ein solcher einzelner Auflagebereich 42a auf der Seite des Fluideinlasses 16 oberhalb der Austrittsöffnung zwischen dem Ventilplenum 18 und dem Fluidauslass 16 ausgebildet.

Die Oberseite 46 der Auflage 42 ist uneben ausgebildet, so dass die Oberseite 46 der Auflage 42 in einer gedachten gewölbten Fläche liegt, es also es keine gedachte Ebene gibt, die die Oberseite 46 der Auflage 42 vollständig enthält. Die gedachte gewölbte Fläche überdeckt eine Oberseite des Ventilkörpers 12. Sie liegt insbesondere orthogonal zu der Fluideinlassachse A, die zugleich eine Längsachse des Ventilkörpers 12 bildet. Wegen der unebenen Form der Oberseite 46 der Auflage 42 würde eine ebene Fläche, die man auf die Oberseite 46 der Auflage aufzulegen versucht, nicht die gesamte Oberseite 46 der Auflage 42 berühren. Vielmehr würden nur einzelne Stellen der ebenen Fläche Kontakt mit der Oberseite 46 der Auflage 42 haben. Daher liegt, jedenfalls im nicht in ein Beatmungsgerät 100 eingebauten Zustand des Expirationsventils 10 die ebene Ventilmembran 20 nicht entlang ihres gesamten Umfangs an der Oberseite 46 der Auflage 42 auf. Es gibt vielmehr einzelne Berührungsstellen zwischen Ventilmembran 20 und Auflage entlang des Umfangs der Ventilmembran 20. Die Unebenheit der Oberseite der Auflage 42 ist nun so ausgebildet, dass erst dann, wenn das Expirationsventil 10 in das Beatmungsgerät 100 eingebaut ist und der Ventilkörper 12 unter Spannung steht, so dass er sich deformiert, die Oberseite 46 der Auflage 42 in einer ebenen Fläche zu liegen kommt und die Ventilmembran 20 nun mit ihrer Anlagefläche flächig auf der Oberseite 46 der Auflage 42 aufliegt.

Der Ventilkörper 12 weist wenigstens eine Montagelasche 50 auf, die zum Eingriff mit einem bajonettartigen Expirationsventilanschluss eines Beatmungsgeräts (100) ausgebildet ist. Das Beatmungsgerät weist dann ein Gegeneingriffselement auf, mit dem die Montagelasche 50 beim Einbau des Expirationsventils 10 in Eingriff gelangt, um das Expirationsventil 10 am Beatmungsgerät 100 zu halten. Ein solcher Bajonettverschluss hat die Eigenschaft, dass beim Verriegeln des Expirationsventils 10 am Beatmungsgerät 100 Spannkräfte entstehen, die den Ventilkörper 12 etwas deformieren.

Der Ventilkörper 12 weist wenigstens zwei Montagelaschen 50 auf, die einander zugeordnet sind. Die Montagelaschen 50 sind am radial äußeren Rand des Ventilkörpers 12 um einen Umfang des Ventilkörpers 12 herum angeordnet und stehen in radialer Richtung vom jeweiligen radial äußeren Rand des Ventilkörpers 12 hervor, um jeweilige Eingriffsflächen 52 mit einem entsprechenden Gegeneingriffselement am Beatmungsgerät 100 zu bilden. Bringt man die Montagelaschen 50 in Eingriff mit ihren jeweiligen am Beatmungsgerät 100 vorgesehenen Gegeneingriffselementen, wird der Ventilkörper 12 auf den jeweils zugeordneten Seiten, an denen sich die Montagelaschen 50 befinden, zum Beatmungsgerät 100 hin geführt. Im Zuge dieser Zwangsbewegung entsteht eine Spannung im Ventilkörper 12, und die obere Kante 48 des Ventilkörpers 12 biegt sich auf den Seiten, an denen die Montagelaschen 50 angeordnet sind, zum Beatmungsgerät 100 hin. Die unebene Ausgestaltung der Oberseite 46 der am Ventilkörper 12 vorgesehenen Auflage 42 für die Ventilmembran 20 soll diese Verbiegung des Ventilkörpers 12 ausgleichen. Nach Ineingriffbringen der Montagelaschen 50 mit dem jeweiligen Gegeneingriffselement am Beatmungsgerät 100 soll die Oberseite 46 der Auflage 42 flach sein, also in einer Ebene liegen. Das beiden Montagelaschen 50 liegen einander in einem Winkel von 180° zueinander gegenüber, d.h. auf gegenüberliegenden Seiten des Ventilkörpers 12 über das Ventilplenum 18 hinweg.

Die Oberseite 46 der Auflage 42 liegt in einer sich bezogen auf das Ventilplenum 18 nach außen gewölbten gedachten Fläche, die die Oberseite des Ventilkörpers 12 überdeckt. Insbesondere liegt die nach außen gewölbte gedachte Fläche bezogen das Ventilplenum 18 weiter außen als eine gedachte Ebene durch wenigstens zwei verschiedene Punkte auf der Oberseite 46 der Auflage 42 an Seiten, an denen Montagelaschen 50 vorhanden sind (also auf der linken und rechten Seite in Fig. 4). Die Oberseite 46 der Auflage 42 liegt dabei in einer konvex gewölbten gedachten Fläche, so dass eine zwei in der gedachten Fläche liegende Punkte verbindende Linie durch das Ventilplenum 18 verläuft. Dabei wölbt sich die Oberseite 46 der Auflage 42 ausgehend von den beiden Montagelaschen 50 nach außen. An den Stellen, an denen sich die Montagelaschen 50 befinden, wölbt sich die Oberseite 46 der Auflage 42 gar nicht nach außen, wohingegen sie sich in der Mitte zwischen den beiden Montagelaschen 50 (in Fig. 4 entlang einer Linie von oben nach unten orthogonal zu einer Verbindung zwischen den beiden Montagelaschen 50) am meisten nach außen auswölbt. Dies trägt der Tatsache Rechnung, dass die Deformierung des Ventilkörpers 12 beim Befestigen des Expirationsventils 10 am Beatmungsgerät 100 dort am stärksten ist, wo sich die Montagelaschen 50 befinden. An dieser Stelle wird die obere Kante 48 des Ventilkörpers 12 am stärksten nach oben gebogen.

In dem gezeigten Ausführungsbeispiel ist die gewölbte gedachte Fläche eine Mantelfläche eines Zylinders, wobei die Achse des Zylinders orthogonal zu einer Verbindungslinie zwischen den beiden zugeordneten Montagelaschen 50 ist.

Wegen der beim Befestigen des Expirationsventils 10 an Stellen, an den Montagelaschen 50 sind, auf den den Ventilkörper 12 ausgeübten Spannung biegt sich auch die obere Kante 48 des Ventilkörpers 12 in derselben Weise an diesen Stellen zu einer Gegenseite am Beatmungsgerät 100 hin. Zur Kompensation dieses Effektes ist es günstig, auch den Ventilkörper 12, genauer gesagt, die obere Kante 48 des Ventilkörpers 12, in entsprechender Weise auszubilden wie die Auflage 42 für die Ventilmembran 20. Die obere Kante 48 des Ventilkörpers soll 12 nach Befestigen des Expirationsventils 10 am Beatmungsgerät 100 an einer Gegenfläche des Beatmungsgeräts 100 anliegen und zwar in einer möglichst flächigen Weise, um eine möglichst dichte Anlage der Ventilmembran 20 zu gewährleisten. Deshalb weist der Ventilkörper 12 eine obere Kante 48 auf, welche in einer sich bezogen auf das Ventilplenum 18 nach außen gewölbten gedachten Fläche liegt. Die bereits dargelegten Überlegungen für die Oberseite 46 der Auflage 42 gelten für die Ausgestaltung der oberen Kante 48 des Ventilkörpers 12 in entsprechender Weise. Insbesondere liegt die obere Kante 48 des Ventilkörpers 12 in einer sich bezogen auf das Ventilplenum 18 konvex gewölbten gedachten Fläche und wölbt sich ausgehend von der wenigstens einen Montagelasche 50 aus nach außen. Dabei ist die Auswölbung der oberen Kante 48 des Ventilkörpers 12 in einem zwischen den beiden Montagelaschen 50 liegenden Bereich größer als in einem einer der beiden Montagelaschen 50 benachbarten Bereich.

Die unebene Form der Oberseite 46 der Auflage 42 und die unebene Form der oberen Kante 48 des Ventilkörpers 12 sind aufeinander abgestimmt. Das ist sinnvoll, denn es soll für beide erreicht werden, dass nach Befestigen des Expirationsventils 10 am Beatmungsgerät 100 sowohl die Oberseite 46 der Auflage 42 als auch die obere Kante 48 des Ventilkörpers 12 in einer ebenen Fläche liegt. Insbesondere wölben sich die Oberseite 46 der Auflage 42 und die obere Kante 48 des Ventilkörpers 12 zueinander ausgerichtet nach außen, wobei die Orte minimaler Auswölbung und maximaler Auswölbung aufeinander abgestimmt sind, insbesondere in Bezug auf eine das Ventilplenum 18 überdeckende Ebene an derselben Stelle liegen. Es ist sogar so, dass die Oberseite 46 der Auflage 42 und die obere Kante 48 des Ventilkörpers 12 sich in etwa gleichermaßen auswölben und insbesondere in in zueinander parallelen gedachten Flächen liegen.

Jeder der Montagelaschen 50 weist eine Eingriffsfläche 52 zum Eingriff mit einem am Beatmungsgerät 100 vorgesehenen Gegeneingriffselement auf, wobei die Eingriffsfläche 52 mit einer Einlaufschräge 52a versehen ist, die zu der Auswölbung der Oberseite der Anlage 42 abgestimmt ist. Wie bei einem Bajonetteingriff üblich, wird die Eingriffsfläche 52 in der Regel im Wesentlichen parallel zu einer oberen Kante der Montagelasche ausgebildet sein. In ihrem vorderen Bereich ist die Einlaufschräge 52a vorgesehen. Im Bereich der Einlaufschräge 52a verläuft die Eingriffsfläche 52 dann in einem Winkel, insbesondere einem spitzen Winkel, zu der oberen Kante der Montagelasche 50. Der Winkel ist so gewählt, dass eine Dicke zwischen Eingriffsfläche 52 und oberer Kante der Montagelasche 50 mit zunehmendem Abstand vom Beginn der Eingriffsfläche 52a zunimmt. Damit lässt sich eine gewisse Spannung beim Ineingriffbringen der Montagelasche 50 mit dem Gegeneingriffselement am Beatmungsgerät 100 erzeugen, was zu einem gewissen Kraftschluss und damit einem festen und sicheren Sitz des Expirationsventils 10 am Beatmungsgerät 100 verhilft. Die Einlaufschräge 52a ist so ausgebildet, dass beim Einspannen der Montagelasche 50 am Beatmungsgerät 100 eine Vorspannung erzeugt wird, die auf die obere Kante 48 des Ventilkörpers auf der Seite der Montagelasche 50 einwirkt. Damit einher geht eine gewisse Deformation des Ventilkörpers 12 auf der der Montagelasche 50 zugewandten Seite gegenüber Seiten oder Stellen weiter abseits der Montagelasche 50. Insbesondere wird die obere Kante 48 des Ventilkörpers 12 durch Bewegung des Gegeneingriffselements entlang der Einlaufschräge 52a näher zum Beatmungsgerät 100 hin geführt. Andererseits liegen weiter von der Montagelasche 50 entfernte Stellen bereits an einer Kontaktfläche des Beatmungsgeräts 100 an und können sich nicht weiter annähern. Im Ergebnis entsteht eine Verbiegung der oberen Kante 48 des Ventilkörpers 12, wobei die Verbiegung zu der Montagelasche 50 hin immer stärker wird. Dadurch entsteht eine Vorspannung im Ventilkörper 12, die mit zunehmender Bewegung des Gegeneingriffselements entlang der Einlaufschräge 52a zunimmt. Die Einlaufschräge 52a ist so gewählt, dass beim Einspannen eine Vorspannung erzeugt wird, die gerade groß genug ist, um die obere Kante 48 des Ventilkörpers 12 im eingespannten Zustand eben zu machen, d.h. dass die obere Kante 48 des Ventilkörpers 12 im eingespannten Zustand in einer das Ventilplenum 18 überdeckenden Ebene liegt. Dann liegt die obere Kante 48 des Ventilkörpers 12 flächig an einer Gegenfläche des Beatmungsgeräts 100 an. Gleichermaßen verbiegt sich auch die integral mit dem Ventilkörper 12 ausgebildete Auflage 42 für die Ventilmembran 20. Damit die Oberseite 46 dieser Auflage 42 im am Beatmungsgerät 100 befestigten Zustand des Expirationsventils 10 ebenfalls eben ist, ist es günstig, wenn die Oberseite 46 der Auflage 42 in demselben Maße uneben ist wie die obere Kante 48 des Ventilkörpers 12. Die Einlaufschräge 52a der Montagelasche 50 ist demgemäß auf die Auswölbung der Auflage 42 abgestimmt.

Um das Ende der Einlaufschräge 52a möglichst gut zu definieren und damit die Vorspannung für den Ventilkörper 12 möglichst genau einstellen zu können, kann vorgesehen sein, dass die Eingriffsfläche 52 einen auf die Einlaufschräge 52a folgenden nicht abgeschrägten Bereich 52b aufweist. Im nicht abgeschrägten Bereich 52b ist die Eingriffsfläche 52 parallel zu der oberen Kante der Montagelasche 50. Eine einmal erreichte Vorspannung und Verbiegung des Ventilkörpers 12 änderst sich damit nicht mehr, wenn das Gegeneingriffselement den nicht abgeschrägten Bereich 52b erreicht. Damit kann die Vorspannung und Verbiegung gut reproduzierbar eingestellt werden, weil es nicht genau darauf ankommt, bis zu welcher Stelle des nicht abgeschrägten Bereichs 52b die Montagelasche 50 gelangt. Zudem ist noch die Eingriffsfläche 52 an ihrem Ende mit einem Anschlag 54 versehen, der ein Ende der Ineingriffbewegung zwischen Montagelaschen 50 und Beatmungsgerät 100 festlegt.

Das beschriebene Expirationsventil 10 kann als ein Einwegbauteil ausgebildet sein, d.h. so ausgebildet sein, dass es nur einmalig einsetzbar ist und nach Gebrauch entsorgt werden muss. Solche Expirationsventile sind billig herzustellen und erfüllen doch die geforderten Hygienestandards. Alternativ kann das Expirationsventil 10 auch so ausgebildet sein, dass ein mehrmalige Verwendung möglich ist. Hierfür wäre die Gestaltung des Expirationsventils 10 so auszuführen, dass es nach Gebrauch desinfizierbar ist, beispielsweise durch Autoklavieren. Das Expirationsventil ist dann autoklavierfest auszulegen.

## Patentansprüche

1. Expirationsventil (10) eines Beatmungsgeräts (100), aufweisend
einen Ventilkörper (12), in dem ein Fluideinlass (14) und ein Fluidauslass (16) für Atemluft eines Patienten ausgebildet sind,
wobei der Ventilkörper (12) wenigstens eine um den Fluideinlass (14) herum ausgebildete Versteifungsrippe (36, 38) aufweist;
wobei die wenigstens eine Versteifungsrippe (36, 38) bezogen auf einen Rand (26) des Fluideinlasses (14) nach außen weg verläuft;
wobei der Ventilkörper (12) ein topfförmiges Ventilplenum (18) bildet, in das der Fluideinlass (14) mündet, wobei das topfförmige Ventilplenum wenigstens zum Teil an seiner Unterseite von dem Ventilkörper (12) umschlossen ist;
wobei der Ventilkörper (12) einen das Ventilplenum (18) wenigstens zum Teil einschließenden Ventilboden (24) bildet, welcher sich tellerartig zwischen dem Fluideinlass (14) und einem Außenrand des Ventilplenums (18) erstreckt,
**dadurch gekennzeichnet, dass**
sich der Ventilboden (24) flach bis flach konisch zwischen seinem inneren Rand (26) und seinem äußeren Rand erstreckt; und
die wenigstens eine Versteifungsrippe eine erste Versteifungsrippe (36) umfasst, die von dem Rand (26) des Fluideinlasses (14) entlang des Ventilbodens (24) nach außen verläuft.

2. Expirationsventil (12) nach Anspruch 1, wobei der Ventilkörper (12) aus Kunststoffmaterial hergestellt ist.

3. Expirationsventil (10) nach Anspruch 1 oder 2, wobei der Ventilkörper (12) ein Spritzgussteil ist.

4. Expirationsventil (10) nach einem der Ansprüche 1 bis 3, wobei die wenigstens eine Versteifungsrippe (36, 38) integral mit dem Ventilkörper (12) ausgebildet ist.

5. Expirationsventil (10) nach einem der Ansprüche 1 bis 4, wobei der Ventilkörper (12) einen Fluideinlassteil (28)aufweist, in dem der Fluideinlass (14) ausgebildet ist.

6. Expirationsventil (10) nach Anspruch 5, wobei der Fluideinlassteil (28) einen ersten zapfenartigen Ansatz ausbildet, wobei der zapfenartige Ansatz zylindrisch oder konisch geformt ist.

7. Expirationsventil (10) nach einem der Ansprüche 1 bis 6, wobei der Fluideinlass (14) in einem zentralen Bereich des Ventilplenums (18) angeordnet ist.

8. Expirationsventil (10) nach einem der Ansprüche 1 bis 7, wobei der Fluidauslass (16) bezogen auf den Fluideinlass (14) in radialer Richtung weiter außen angeordnet ist.

9. Expirationsventil (10) nach einem der Ansprüche 1 bis 8, wobei der Ventilboden (24) von einem eine Fluideintrittsfläche definierenden Rand des Fluideinlasses ausgeht.

10. Expirationsventil (10) nach Anspruch 9, wobei der Ventilboden (24) ausgehend von einer Innenseite des Fluideinlasses (14) am Rand (26) der Fluideintrittsfläche (22) auf seiner dem Ventilplenum (18) zugewandten Seite eine abgerundete Kontur aufweist.

11. Expirationsventil (10) nach einem der Ansprüche 1 bis 10, wobei der Ventilboden (24) sich in einem Bereich um den Fluideinlass (14) herum in axialer Richtung auswölbt.

12. Expirationsventil (10) nach einem der Ansprüche 1 bis 11, wobei der Ventilboden (24) einen Fluidströmungsweg zwischen dem Fluideinlass (14) und dem Fluidauslass (16) definiert, dessen Querschnitt sich zum Fluidauslass (16) hin erweitert.

13. Expirationsventil (10) nach Anspruch 12, aufweisend eine axiale Auswölbung (32), welche eine sich um den Umfang des Fluideinlasses (14) herum verändernde Tiefe hat.

14. Expirationsventil (10) nach Anspruch 13, wobei die Tiefe der Auswölbung (32) in Umfangsrichtung zu dem Fluidauslass (16) hin zunimmt.

15. Expirationsventil (10) nach Anspruch 13 oder 14, wobei die Tiefe der Auswölbung (32) in Umfangsrichtung auf einer dem Fluidauslass (16) gegenüber liegenden Seite am kleinsten ist.

16. Expirationsventil (18) nach einem der Ansprüche 13 bis 15, wobei die Tiefe der Auswölbung (32) in Umfangsrichtung auf der zum Fluidauslass (16) hin gelegenen Seite am größten ist.

17. Expirationsventil (10) nach einem der Ansprüche 1 bis 16, wobei die wenigstens eine Versteifungsrippe eine zweite Versteifungsrippe (38) umfasst, die von dem äußeren Rand des Ventilbodens (24) entlang des Ventilbodens (24) nach innen verläuft.

18. Expirationsventil (10) nach einem der Ansprüche 1 bis 17, wobei der Ventilkörper (12) einen Fluidauslassteil (30) bildet, in dem der Fluidauslass (16) ausgebildet ist.

19. Expirationsventil (10) nach Anspruch 18, wobei der sich auswölbende Bereich (32) des Ventilbodens (24) in den Fluidauslassteil (30) übergeht.

20. Expirationsventil nach einem der vorangehenden Ansprüche, ferner aufweisend eine dem Fluideinlass zugeordnete Ventilmembran, die mindestens zwischen einer den Fluideinlass verschließenden Stellung und einer den Fluideinlass öffnenden Stellung verlagerbar ist.

21. Expirationsventil (10) nach einem der vorangehenden Ansprüche, wobei der Ventilkörper (12) ferner eine Auflage (42) aufweist, an deren Oberseite die Ventilmembran (20) mit einer an ihrem äußeren Rand ausgebildeten Anlagefläche auflegbar ist, wobei die die Auflage (42) auf ihrer Oberseite (46) uneben ausgebildet ist.

22. Beatmungsgerät (100), aufweisend ein an eine Expirationsluftleitung anschließbares Expirationsventil (10) nach einem der vorangehenden Ansprüche.

## Claims

1. An expiratory valve (10) of a ventilation device (100), comprising
a valve body (12) having a fluid inlet (14) and a fluid outlet (16) for breathing air of a patient formed therein,
said valve body (12) having at least one reinforcing rib (36, 38) formed around the fluid inlet (14);
wherein the at least one reinforcing rib (36, 38) extends outwardly with respect to an edge (26) of the fluid inlet (14);
wherein the valve body (12) forms a cup-shaped valve plenum (18) into which the fluid inlet (14) opens, said cup-shaped valve plenum being at least partly enclosed by the valve body (12) on its bottom side;
wherein the valve body (12) forms a valve bottom (24) which at least partly encloses the valve plenum (18), said valve bottom (24) extending in dish-like manner between the fluid inlet (14) and an outer edge of the valve plenum (18),
**characterized in that**
the valve bottom (24) extends in a flat to flat conical manner between its inner edge (26) and its outer edge; and
that the at least one reinforcing rib comprises a first reinforcing rib (36) extending outwardly from the edge (26) of the fluid inlet (14) along the valve bottom (24).

2. The expiratory valve (10) of claim 1,
wherein the valve body (12) is made of plastics material.

3. The expiratory valve (10) of claim 1 or 2,
wherein the valve body (12) is an injection-molded part.

4. The expiratory valve (10) of any of claims 1 to 3,
wherein the at least one reinforcing rib (36, 38) is formed integrally with said valve body (12).

5. The expiratory valve (10) of any of claims 1 to 4,
wherein the valve body (12) has a fluid inlet portion (28) having the fluid inlet (14) formed therein.

6. The expiratory valve (10) of claim 5,
wherein the fluid inlet portion (28) forms a first pin-like protrusion, said pin-like protrusion having a cylindrical or conical shape.

7. The expiratory valve (10) of any of claims 1 to 6,
wherein the fluid inlet (14) is disposed in a central portion of the valve plenum (18).

8. The expiratory valve (10) of any of claims 1 to 7,
wherein the fluid outlet (16) is disposed further outside in the radial direction with respect to the fluid inlet (14).

9. The expiratory valve (10) of any of claims 1 to 8,
wherein the valve bottom (24) originates from an edge of the fluid inlet defining a fluid entry surface.

10. The expiratory valve (10) of claim 9,
wherein the valve bottom (24), starting from an inside of the fluid inlet (14) at the edge (26) of the fluid entry surface (22), has a rounded contour on its side facing the valve plenum (18).

11. The expiratory valve (10) of any of claims 1 to 10,
wherein the valve bottom (24) bulges in axial direction in a portion around the fluid inlet (14).

12. The expiratory valve (10) of any of claims 1 to 11,
wherein the valve bottom (24) defines a fluid flow path between the fluid inlet (14) and the fluid outlet (16), with the cross-sectional area of said fluid flow path expanding towards the fluid outlet (16).

13. The expiratory valve (10) of claim 12,
comprising an axial bulge (32) having a varying depth around the circumference of the fluid inlet (14).

14. The expiratory valve (10) of claim 13,
wherein the depth of the bulge (32) increases in circumferential direction towards the fluid outlet (16).

15. The expiratory valve (10) of claim 13 or 14,
wherein the depth of the bulge (32) in circumferential direction is smallest on a side opposite the fluid outlet (16).

16. The expiratory valve (10) of any of claims 13 to 15,
wherein the depth of the bulge (32) in circumferential direction is largest on the side towards the fluid outlet (16).

17. The expiratory valve (10) of any of claims 1 to 16,
wherein the at least one reinforcing rib comprises a second reinforcing rib (38) extending inwardly from the outer edge of the valve bottom (24) along the valve bottom (24).

18. The expiratory valve (10) of any of claims 1 to 17,
wherein the valve body (12) defines a fluid outlet portion (30) having the fluid outlet (16) formed therein.

19. The expiratory valve (10) of claim 18,
wherein the bulging portion (32) of the valve bottom (24) merges into the fluid outlet portion (30).

20. The expiratory valve of any of the preceding claims,
further comprising a valve membrane associated with the fluid inlet, which is displaceable at least between a position closing the fluid inlet and a position opening the fluid inlet.

21. The expiratory valve (10) of any of the preceding claims,
wherein the valve body (12) further comprises a support (42) onto the top side of which the valve membrane (20) can be placed with an abutment area formed at the outer edge thereof, wherein the support (42) has an uneven design on its top side (46).

22. A ventilation device (100), comprising an expiratory valve (10) according to any of the preceding claims that is adapted to be connected to an expiration air conduit.

## Revendications

1. Soupape d'expiration (10) d'un respirateur artificiel (100) qui présente :
un corps de soupape (12) dans lequel sont réalisées une entrée destinée à un fluide (14) et une sortie (16) destinée à un fluide pour l'air que l'on respire chez un patient ;
dans laquelle le corps (12) de la soupape présente au moins une nervure de renforcement (36, 38) qui est réalisée autour de l'entrée (14) destinée à un fluide ;
- dans laquelle ladite au moins une nervure de renforcement (36, 38) s'étend vers l'extérieur par rapport à un bord (26) de l'entrée (14) destinée à un fluide ;
dans laquelle le corps (12) de la soupape forme une chambre de soupape (18) présentant une configuration en forme de pot, dans laquelle débouche l'ouverture (14) destinée à un fluide; dans laquelle la chambre de la soupape possédant une configuration en forme de pot est entourée au moins en partie, sur son côté inférieur, par le corps (12) de la soupape ;
dans laquelle le corps (12) de la soupape forme une base de soupape (24) qui renferme la chambre (18) de la soupape au moins en partie, base qui s'étend en formant un plateau entre l'entrée (14) destinée à un fluide et un bord externe de la chambre (18) de la soupape ;
**caractérisée**
**en ce que** la base (24) de la soupape s'étend depuis une position de forme aplatie jusqu'à une position de forme aplatie-conique entre son bord interne (26) et son bord externe ; et
**en ce que** ladite au moins une nervure de renforcement comprend une première nervure de renforcement (36) qui s'étend vers l'extérieur à partir du bord (26) de l'entrée (14) destinée à un fluide le long de la base (24) de la soupape.

2. Soupape d'expiration (10) selon la revendication 1, dans laquelle le corps (12) de la soupape est réalisé à partir d'une matière synthétique.

3. Soupape d'expiration (10) selon la revendication 1 ou 2, dans laquelle le corps (12) de la soupape représente une pièce que l'on obtient par l'intermédiaire d'un moulage par injection.

4. Soupape d'expiration (10) selon l'une quelconque des revendications 1 à 3, dans laquelle ladite au moins une nervure de renforcement (36, 38) est réalisée en une seule pièce avec le corps (12) de la soupape.

5. Soupape d'expiration (10) selon l'une quelconque des revendications 1 à 4, dans laquelle le corps (12) de la soupape présente un élément (28) faisant office d'entrée pour un fluide dans lequel est réalisée l'entrée (14) destinée à un fluide.

6. Soupape d'expiration (10) selon la revendication 5, dans laquelle l'élément (28) faisant office d'entrée pour un fluide forme un premier embout en forme de tourillon ; dans laquelle l'embout en forme de tourillon est réalisé pour prendre une configuration de forme cylindrique ou de forme conique.

7. Soupape d'expiration (10) selon l'une quelconque des revendications 1 à 6, dans laquelle l'entrée (14) destinée à un fluide est disposée dans une zone centrale de la chambre (18) de la soupape.

8. Soupape d'expiration (10) selon l'une quelconque des revendications 1 à 7, dans laquelle la sortie (16) destinée à un fluide est disposée plus vers l'extérieur dans la direction radiale par rapport à l'entrée (14) destinée à un fluide.

9. Soupape d'expiration (10) selon l'une quelconque des revendications 1 à 8, dans laquelle un bord de l'entrée destinée à un fluide, qui définit une surface d'entrée pou un fluide représente le point de départ de la base (24) de la soupape.

10. Soupape d'expiration (10) selon la revendication 9, dans laquelle la base (24) de la soupape dont le point de départ représente un côté interne de l'entrée (14) destinée à un fluide contre le bord (26) de la surface d'entrée (22) pour un fluide présente, sur son côté tourné vers la chambre (18) de la soupape, un contour arrondi.

11. Soupape d'expiration (10) selon l'une quelconque des revendications 1 à 10, dans laquelle la base (24) de la soupape prend une configuration de forme bombée dans la direction axiale dans une zone qui entoure complètement l'entrée (14) destinée à un fluide.

12. Soupape d'expiration (10) selon l'une quelconque des revendications 1 à 11, dans laquelle la base (24) de la soupape définit un chemin d'écoulement pour un fluide entre l'entrée (14) destinée à un fluide et la sortie (16) destinée à un fluide, dont la section transversale s'élargit dans la direction de la sortie (16) destinée à un fluide.

13. Soupape d'expiration (10) selon la revendication 12, qui présente un bombement axial (32) qui présente une profondeur qui se modifie autour de la périphérie de l'entrée (14) destinée à un fluide.

14. Soupape d'expiration (10) selon la revendication 13, dans laquelle la profondeur du bombement (32) augmente dans la direction circonférentielle en direction de la sortie (16) destinée à un fluide.

15. Soupape d'expiration (10) selon la revendication 13 ou 14, dans laquelle la profondeur du bombement (32) est la plus petite dans la direction circonférentielle sur un côté qui est situé à l'opposé de la sortie (16) destinée à un fluide.

16. Soupape d'expiration (10) selon l'une quelconque des revendications 13 à 15, dans laquelle la profondeur du bombement (32) est la plus grande dans la direction circonférentielle sur le côté qui est orienté vers la sortie (16) destinée à un fluide.

17. Soupape d'expiration (10) selon l'une quelconque des revendications 1 à 16, dans laquelle ladite au moins une nervure de renforcement comprend une deuxième nervure de renforcement (38) qui s'étend vers l'intérieur à partir du bord externe de la base (24) de la soupape le long de la base (24) de la soupape.

18. Soupape d'expiration (10) selon l'une quelconque des revendications 1 à 17, dans laquelle le corps (12) de la soupape forme une partie (30) de la sortie destinée à un fluide, dans laquelle la sortie (16) destinée à un fluide est réalisée.

19. Soupape d'expiration (10) selon la revendication 18, dans laquelle la zone (32) qui forme un bombement, de la base (24) de la soupape se transforme pour obtenir la partie (30) de la sortie destinée à un fluide.

20. Soupape d'expiration (10) selon l'une quelconque des revendications précédentes, qui présente en outre une membrane faisant office de soupape qui est attribuée à l'entrée destinée à un fluide, ladite membrane étant à même de se déplacer pour prendre une position choisie entre au moins une position qui ferme l'entrée destinée à un fluide et une position qui ouvre l'entrée destinée à un fluide.

21. Soupape d'expiration (10) selon l'une quelconque des revendications précédentes, dans laquelle le corps (12) de la soupape présente en outre un recouvrement (42) qui peut venir se disposer contre le côté supérieur de la membrane (20) faisant office de soupape avec une surface d'appui réalisée le long de son bord externe ; dans laquelle le recouvrement (42) est réalisé pour prendre une forme inégale sur son côté supérieur (46).

22. Respirateur artificiel (100) qui présente une soupape d'expiration (10) selon l'une quelconque des revendications précédentes, qui peut venir se raccorder à un conduit destiné à l'air que l'on expire.
